(19) Europäisches Patentamt
European Patent Office
Office européen des brevets

(11) **EP 2 474 265 B1**

(12) **EUROPEAN PATENT SPECIFICATION**

(45) Date of publication and mention
of the grant of the patent:
**21.08.2013 Bulletin 2013/34**

(51) Int Cl.:
*A61B 5/1455* *(2006.01)* *A61B 1/06* *(2006.01)*

(21) Application number: **11188217.1**

(22) Date of filing: **08.11.2011**

(54) **Endoscope diagnosis system**

Endoskopisches Diagnosesystem

Système de diagnostic d'endoscope

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.01.2011 JP 2011002950**

(43) Date of publication of application:
**11.07.2012 Bulletin 2012/28**

(73) Proprietor: **Fujifilm Corporation
Minato-ku
Tokyo (JP)**

(72) Inventor: **Saito, Takaaki
Kanagawa (JP)**

(74) Representative: **Klunker . Schmitt-Nilson . Hirsch
Patentanwälte
Destouchesstrasse 68
80796 München (DE)**

(56) References cited:
**EP-A1- 1 302 152 US-A1- 2010 280 322**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

BACKGROUND OF THE INVENTION

The present invention relates to an endoscopic diagnosis system for simultaneously observing the blood level (amount of blood) in the medium to deep layer of a mucous membrane and blood vessels in the superficial layer.

There is conventionally used an endoscope device wherein white light (normal light) emitted from a light source device is guided to the tip of an endoscope to illuminate a region under observation of a subject, and the reflected light is imaged to acquire a normal light image (white light image) in order to perform normal light observation (white light observation). In recent years, there is used an endoscope device wherein a region of a subject under observation is illuminated by narrowband light (special light) having a given wavelength range, and the reflected light thereof, for example, is imaged to acquire a special light image (narrowband light image) in order to perform special light observation in addition to normal light observation.

An endoscope device capable of special light observation can readily visualize biological information, such as a fine structure of a blood vessel newly formed in a mucosa layer or beneath a mucosa layer of a subject's lumen and a visually enhanced site of lesion, which is unobtainable from a normal observation image. When, for example, the site to be observed is a cancer-affected region, illuminating a mucosal tissue with blue narrowband light enables observation of fine blood vessels and a fine structure in the superficial layer of the tissue in greater detail and thus permits diagnosis of a site of lesion with an increased accuracy.

As described above, an endoscope device for narrowband light observation is capable of display whereby thin blood vessels lying in a superficial layer of a mucous membrane are enhanced, and this feature is widely used for endoscopic diagnosis of cancers. On the other hand, an undifferentiated early-stage stomach cancer, for example, tends to extend in a direction lateral to the medium layer of a mucous membrane, and therefore detection of the site of lesion and determination of the affected region are difficult to achieve by the observation of the superficial layer of the mucous membrane as effectively carried out by narrowband light observation.

On the other hand, JP 3559755 B describes an endoscope device whereby narrowband light of red, green, and blue light are allowed to illuminate a subject by frame sequential method, images of the respective wavelength bands of the red, green, and blue light are acquired, whereupon the hemoglobin index IHb, which correlates to hemoglobin in the blood and corresponds to the blood level in the medium to deep layer, is calculated as, for example, log(R/G) to produce a pseudo color image based on the IHb or replace one band image, for example an image of red light, with an IHb image, in order to display the blood level in the medium to deep layer in pseudo color.

Document EP 1302152 discloses the preamble of claims 1 and 11.

SUMMARY OF THE INVENTION

According to JP 3559755 B, however, while the blue-light image acquired by illuminating the subject with the narrowband light of blue light contains information on superficial-layer blood vessels, merely displaying the blue-light image as it is did not permit easy observation of superficial-layer blood vessels. Further, while the hemoglobin index IHb contains information on the blood level in the medium to deep layer, merely displaying the information on the hemoglobin index IHb in pseudo color did not permit easy distinction between a high blood level region and a low blood level region.

An object of the present invention is to provide an endoscopic diagnosis system capable of displaying an endoscopic image permitting easy recognition of both the blood level in the medium to deep layer of a mucous membrane and blood vessels in the superficial layer.

In order to achieve the above-described objects, the present invention provides an endoscopic diagnosis system, comprising:

a white light source for emitting white light;
a first narrowband light source for emitting first narrowband light having a given wavelength range of blue light;
an image sensor including blue, green and red color filters on its light receiving surface and receiving reflected light of the white light and the first narrowband light emitted with a given emission ratio to illuminate a subject from the subject in a narrowband light observation mode to acquire a narrowband light image, an image processor for calculating an enhanced luminance signal Y where a blue image signal B of the narrowband light image is enhanced, calculating a hemoglobin index representing a blood level in a medium to deep layer of a mucous membrane from a green image signal G and a red image signal R of the narrowband light image, calculating an enhanced color difference signal including a red color difference signal Cr and a blue color differences signal Cb enhancing red according to a value of the hemoglobin index, and producing an image signal of an endoscopic image for display from the enhanced luminance signal and the enhanced color difference signal; and
a monitor for displaying an endoscopic image corresponding to the image signal of the endoscopic image for display.

Also, the present invention provides an endoscopic diagnosis system, comprising:

a white light source for emitting white light;

color filters for separating the white light into narrowband light, green light and red light, the narrowband light having a given wavelength range of blue light;

an image sensor for receiving reflected light of the narrowband light having the given wavelength range of the blue light, the green light and the red light emitted by frame sequential method to illuminate a subject and sequentially acquiring a blue narrowband light image, a green normal light image and a red normal light image in a narrowband light observation mode;

an image processor for calculating an enhanced luminance signal Y where an image signal B of the blue narrowband light image is enhanced, calculating a hemoglobin index representing blood level in a medium to deep layer of a mucous membrane from an image signal G of the green normal light image and an image signal R of the red normal light image, calculating an enhanced color difference signal including a red color difference signal Cr and a blue color differences signal Cb where red is enhanced according to a value of the hemoglobin index, and producing an image signal of an endoscopic image for display from the enhanced luminance signal and the enhanced color difference signal; and

a monitor for displaying an endoscopic image corresponding to the image signal of the endoscopic image for display.

According to the present invention, the blood vessels in the superficial layer can be placed in high contrast by enhancing the blue image signal B of the narrowband light image. Further, the blood level in the medium to deep layer of a mucous membrane can be represented in gradation of red in an image by enhancing the red according to the value of hemoglobin index. Thus, simultaneously displaying the information on a mucous membrane medium-to-deep layer blood amount distribution and enhanced superficial-layer blood vessels in endoscopic observation compensates for a disadvantage of the narrowband light observation that discovery of a site of lesion or determination of the range of an affected region is difficult with a mere inspection into the superficial layer of a mucous membrane and enables improved diagnosis of a region affected by a tumor including the undifferentiated type.

BRIEF DESCRIPTION OF THE DRAWINGS

Fig. 1 is an external view of a first embodiment representing a configuration of the endoscopic diagnosis system of the invention.

Fig. 2 is a block diagram representing an internal configuration of the endoscopic diagnosis system of Fig. 1.

Fig. 3 is a graph illustrating an emission spectrum of a blue laser beam emitted from a blue laser light source and light obtained through wavelength conversion of blue laser beam by a fluorescent body.

Fig. 4 is a graph illustrating spectral transmittances of red, green, and blue filters.

Fig. 5 is a block diagram representing an internal configuration of a narrowband light image processor of Fig. 1.

Fig. 6 is an example of conversion table representing a relationship between hemoglobin index and color-difference signal.

Fig. 7 is a block diagram of a second embodiment illustrating an internal configuration of the endoscopic diagnosis system shown in Fig. 1.

DETAILED DESCRIPTION OF THE INVENTION

The endoscopic diagnosis system according to the present invention will be described in detail based on the preferred embodiments illustrated in the attached drawings.

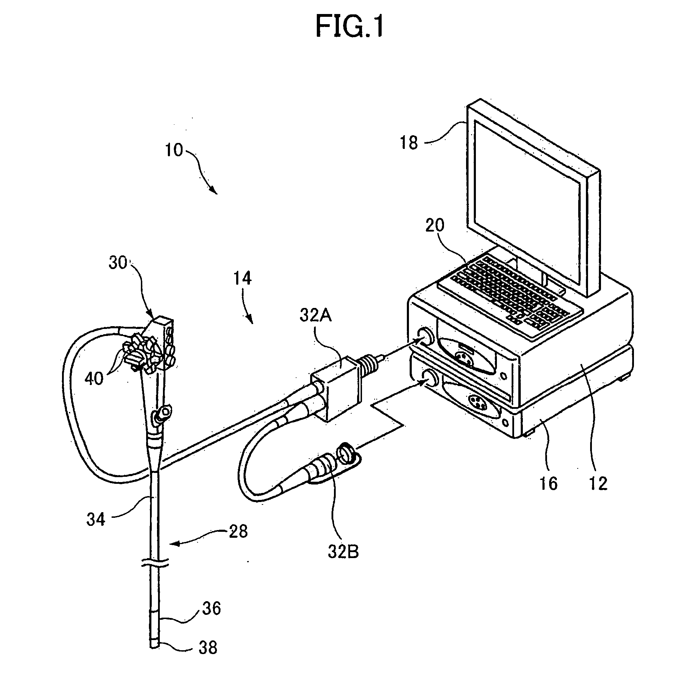

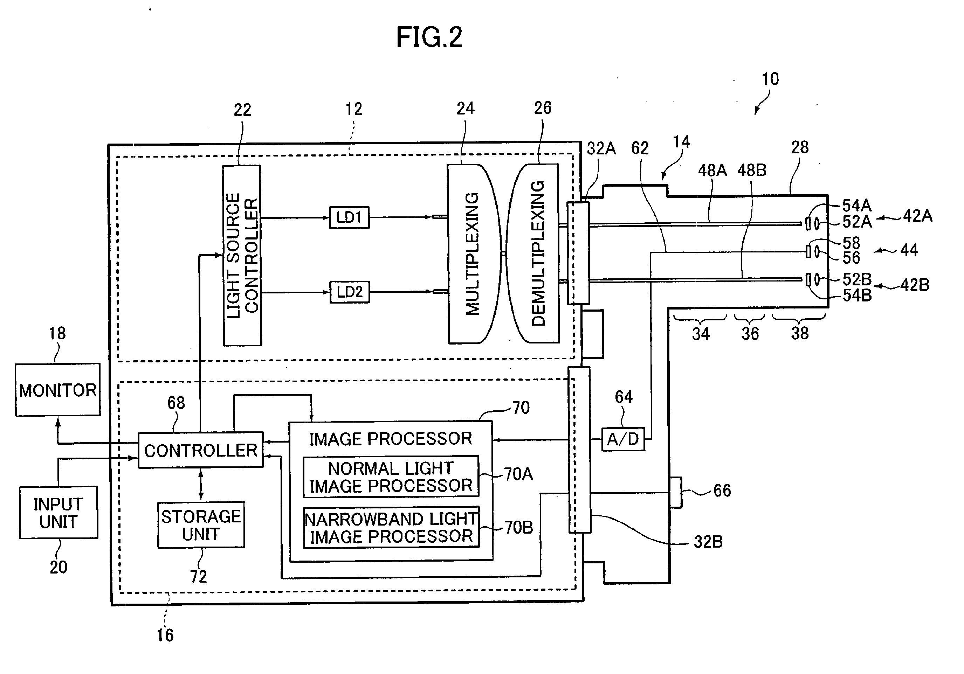

Fig. 1 is an external view of a first embodiment representing a configuration of the endoscopic diagnosis system according to the invention; Fig. 2 is a block diagram representing an internal configuration thereof. A endoscopic diagnosis system 10 illustrated in these figures comprises a light source device 12 for generating light having a given range of wavelength; an endoscope device 14 for guiding light emitted from the light source device 12 to illuminate a subject's region under observation with the illumination light and imaging the reflected light from the subject; a processor 16 for image-processing the image acquired by the endoscope device 14 and outputting an endoscopic image; a monitor 18 for displaying the endoscopic image outputted from the processor 16; and an input unit 20 for receiving input operations.

The endoscopic diagnosis system 10 is capable of normal light (white light) observation mode for illuminating the subject with normal light (white light) and imaging the reflected light thereof to display (observe) a normal light image (white light image) and special light observation mode (narrowband light observation mode) for illuminating the subject with special light (narrowband light) and imaging the reflected light to display a special light image (narrowband light image). The observation mode is switched as appropriate according to an instruction entered by a selector switch 66 of the endoscope device 14 or the input unit 20.

The light source device 12 comprises a light source controller 22, two kinds of laser light sources LD1, LD2 for emitting laser beams having different wavelength ranges, a combiner (multiplexer) 24, and a coupler (demultiplexer) 26.

According to this embodiment, the laser light sources LD1, LD2 emit narrowband light beams having given wavelength ranges of blue light with central wavelengths of 405 nm and 445 nm (e.g., central wavelength +/- 10 nm), respectively. The laser light source LD1 is provided to acquire a narrowband light image; the laser light source LD2 emits excitation light for normal light observation to generate white light (pseudo white light) from a fluorescent body described later.

The white light source (normal light source) for generating white light is not limited to a light source using a combination of excitation light and fluorescent body and may be any light source to generate white light, including, for example, a xenon lamp, a halogen lamp, and a white LED (light emitting diode). The laser beams emitted from the laser light sources LD1, LD2 are not limited in wavelength to the above; laser beams capable of serving the same purpose may be selected as appropriate. The laser beam emitted from the laser light source LD1, for example, is preferably narrowband light having a given wavelength range contained within a wavelength range of blue light of 395 nm to 455 nm for which the hemoglobin in the blood has a high light absorption coefficient. Where the white light source comprises the laser light source LD2 that emits a laser beam having a wavelength range of 445 nm +/- 10 nm and a fluorescent body as in this embodiment, the light for acquiring a narrowband light image may be a laser beam having a central wavelength of 445 nm in lieu of a laser beam having a central wavelength of 405 nm emitted from the laser light source LD1. Thus, the laser light source LD1 may be omitted and costs can be advantageously reduced.

The on/off control and light amount control of the laser light sources LD1, LD2 are made independently between these light sources by the light source controller 22 controlled by a controller of the processor 16 described later, and the emission timing and the emission amount ratio can be freely varied.

The laser light sources LD1, LD2 may be constituted using, among others, broad area type InGaN-based laser diodes as well as InGaNas-based laser diodes and GaNas-based laser diodes.

The light source controller 22 turns the laser light source LD1 off and turns the laser light source LD2 on in the normal light observation mode. The light source controller 22 turns on both the laser light sources LD1 and LD2 in the narrowband light observation mode.

The laser beams emitted from the laser light sources LD1, LD2 are passed through condenser lenses (not shown) to enter their respective optical fibers, combined by the combiner 24 and divided into two channels of light beams by the coupler 26 before being transmitted to a connector unit 32A. The combiner 24 and the coupler 26 are composed of, for example, a half mirror or a reflection mirror. The configuration is not limited this way; the laser beams from the laser light sources LD1, LD2 may be directly transmitted to the connector unit 32A in lieu of through the combiner 24 and the coupler 26.

The endoscope device 14 is an electronic endoscope instrument comprising an optical system for illumination for emitting two channels (two beams) of illumination light from the tip of the endoscope insertion section inserted into the inside of the subject's body and one channel (one image sensor) of optical imaging system for acquiring an endoscopic image of the region under observation. The endoscope device 14 comprises the endoscope insertion section 28, an operating unit 30 for bending the tip of the endoscope insertion section 28 and performing an operation for observation, and connectors 32A, 32B for detachably connecting the endoscope device 14 to the light source device 12 and the processor 16.

The endoscope insertion section 28 comprises a flexible portion 34 having a flexibility, a bending portion 36, and a tip 38 (also referred to below as tip of the endoscope).

The bending portion 36 is provided between the flexible portion 34 and the tip 38 and is so configured as to be bendable by rotating an angle knob 40 provided on the operating unit 30. The bending portion 36 can be bent in any direction and to any angle according to, for example, the subject's site for which the endoscope device 14 is used, so that the endoscope tip portion 38 may be directed toward a desired site for observation.

At the tip end surface of the endoscope tip portion 38 are provided two channels of illumination windows 42A, 42B for emitting light to a region under observation and one channel of observation window 44 for imaging the reflected light from the region under observation.

On the inside of the illumination window 42A is provided an optical fiber 48A. The optical fiber 48A extends from the light source device 12 through the connector unit 32A to the scope tip portion 38. The optical fiber 48A has at its tip portion (the end thereof closer to the illumination window 42A) a fluorescent body 54A, and beyond the fluorescent body 54A is provided an optical system including, for example, a lens 52A. Similarly, on the inside of the illumination window 42B is provided an optical fiber 48B including, for example, a fluorescent body 54B and a lens 52B at the tip portion.

Fluorescent bodies 54A, 54B comprise a plurality of kinds of fluorescent substances that emit green to yellow light when excited upon absorbing part of the blue laser beam emitted from the laser light source LD2 (e.g., YAG-based fluorescent substance or a fluorescent substance such as BAM ($BaMgAl_{10}O_{17}$)). When the excitation light for normal light observation illuminates the fluorescent bodies 54A, 54B, the green to yellow excited luminescence light (fluorescence) emitted from the fluorescent bodies 54A, 54B is allowed to blend with part of the blue laser beam that is passed without being absorbed by the fluorescent bodies 54A, 54B to generate white light (pseudo white light).

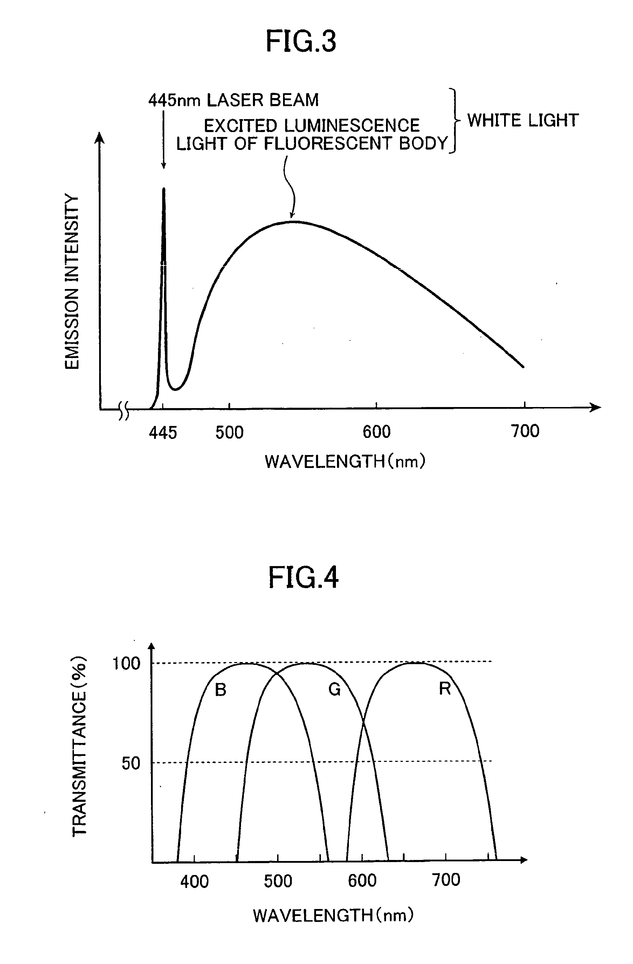

Fig. 3 shows a graph illustrating an emission spectrum of a blue laser beam emitted from the blue laser light source and of light obtained through wavelength conversion of the blue laser beam by the fluorescent body. The blue laser beam emitted from the laser light source LD2 is represented by an emission line having a central wavelength of 445 nm; excited luminescence light excited by the blue laser beam and emitted from the fluorescent bodies 54A, 54B has a spectral intensity distribution such that the light emission intensity increases in a wavelength range of about 450 nm to 700 nm. The excited luminescence light and the blue laser beam combines to produce pseudo white light as described above.

For the purpose of the invention, the white light is not limited to light containing strictly all the wavelength components of visible light but need only contain light having a specific wavelength range such as, for example, light having reference colors such as red, green, and blue, as well as the above pseudo white light. Thus, the white light herein broadly also includes, for example, light containing wavelength components corresponding to green to red light and light containing wavelength components corresponding to blue to green light.

Where a laser beam having a central wavelength of 405 nm is emitted to the fluorescent bodies 54A, 54B, the light intensities of the excited luminescence light emitted from the fluorescent bodies 54A, 54B are a fraction of the light intensities when a laser beam having a central wavelength of 445 nm is emitted to the fluorescent bodies 54A, 54B. In other words, when a laser beam having a central wavelength of 405 nm is emitted to the fluorescent bodies 54A, 54B together with a laser beam having a central wavelength of 445 nm, the fluorescent bodies 54A, 54B scarcely emit excited luminescence light caused by the laser beam having a central wavelength of 405 nm.

In this embodiment, therefore, when a laser beam having a central wavelength of 445 nm, which is excitation light for normal light observation, and a laser beam having a central wavelength of 405 nm, which is narrowband light for blood vessel observation, are allowed to illuminate simultaneously, both light are combined, and a combined laser beam is allowed to illuminate the fluorescent bodies 54A, 54B. One channel of optical system for illumination, for example, may have no fluorescent bodies, and a laser beam having a central wavelength of 405 nm may be guided by an optical fiber having no fluorescent bodies to illuminate a subject.

The optical system for illumination comprising the illumination window 42A and the optical system for illumination comprising the illumination window 42B have an equivalent configuration and operations, so that the illumination windows 42A, 42B basically emit equivalent illumination light simultaneously. The illumination windows 42A, 42B may emit different illumination light. Provision of optical systems for illumination for emitting two channels of illumination light is not essential; optical systems for illumination for emitting one or four channels of illumination light, for example, are capable of performing an equivalent function.

On the inside of the observation window 44 is installed an optical system including, for example, an object lens unit 56 for introducing image light from the subject's region under observation and, further behind the object lens unit 56 is installed an image sensor 58 (first image sensor) such as a CCD (Charge Coupled Device) image sensor and a CMOS (Complementary Metal-Oxide Semiconductor) image sensor for acquiring image information on the subject's region under observation.

The image sensor 58 receives light from the object lens unit 56 with a light receiving surface (imaging surface), photoelectrically converts the received light into an imaging signal (analog signal), and outputs the imaging signal. The receiving surface of the image sensor 58 is provided with red (about 580 nm to 760 nm), green (about 450 nm to 630 nm), and blue (about 380 nm to 510 nm) color filters having spectral transmittances dividing a wavelength range of about 370 nm to 720 nm of visual light into three ranges as illustrated in Fig. 4; a red pixel, a green pixel, and a blue pixel form one set of pixels, and a plurality of sets of pixels are arranged in the form of matrix.

The light emitted from the light source device 12 and guided through the optical fibers 48A, 48B is emitted from the endoscope tip portion 38 toward the subject's region under observation. The region under observation illuminated by the illumination light is imaged on the light receiving surface of the image sensor 58 through the object lens unit 56 and undergoes photoelectric conversion by the image sensor 58 to obtain an image. The image sensor 58 outputs imaging signals (analog signals) of the subject's imaged region under observation.

Imaging signals (analog signals) of images (normal light image and narrowband light image) outputted from the image sensor 58 travel through a scope cable 62 to enter an A/D converter 64. The A/D converter 64 converts the imaging signals (analog signals) supplied from the image sensor 58 into image signals (digital signals). The image signals obtained through the conversion pass through the connector unit 32B to enter an image processor of the processor 16.

The operating unit 30 and the endoscope insertion section 28 contain therein, a forceps channel for inserting, for example, a tissue collecting tool; air supply/water supply channels, and other channels, not shown.

The processor 16 comprises the controller 68, the image processor 70, and a storage unit 72. The controller 68 is connected to the monitor 18 and the input unit 20. The processor 16 controls the light source controller 22 of the light source device 12 according to an instruction inputted from the selector switch 66 of the endoscope device 14 and the input unit 20 and image-processes an image signal inputted from the endoscope device 14 to produce and output a display image to the monitor 18.

The controller 68 controls the operations of the image processor 70 and the light source controller 22 of the light source device 12 according to instructions, such as an observation mode instruction, given by the selector switch 66 of the endoscope device 14 and the input unit 20.

The image processor 70 performs a given image processing on the image signal entered from the endoscope device 14 under the control by the controller 68 according to the observation mode depending on the kinds of images including a normal light image and a narrowband light image. The image signal processed by the image processor 70 is supplied to the controller 68, which produces an endoscopic observation image from this processed image together with other information. The endoscopic observation image is displayed on the monitor 18 and, where necessary, stored in the storage unit 72 composed of a memory or a storage device.

The image processor 70 comprises a normal light image processor 70A and a narrowband light image processor 70B. The normal light image processor 70A and the narrowband light image processor 70B perform given image processing suited to respective endoscopic images on the image signals of the normal light image and the narrowband light image, respectively, in the normal light observation mode and the narrowband light observation mode to output (produce) a normal light image signal and a narrowband light image signal for display.

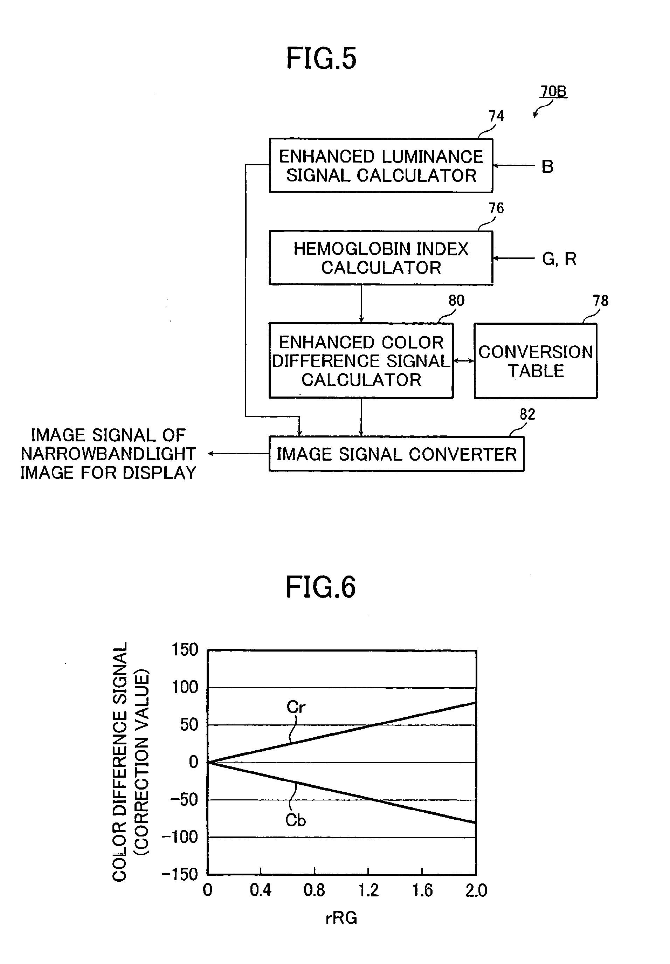

As illustrated in Fig. 5, the narrowband light image processor 70B comprises an enhanced luminance signal calculator 74, a hemoglobin index calculator 76, a conversion table 78, an enhanced color difference signal calculator 80, and an image signal converter 82.

The enhanced luminance signal calculator 74 calculates an enhanced luminance signal where an image signal B of blue pixels of the narrowband light image is enhanced. In this embodiment, the enhanced luminance signal calculator 74 calculates an enhanced luminance signal Y by weighting a blue image signal B and a green image signal G with a given ratio.

The hemoglobin index calculator 76 calculates the hemoglobin index rRG representing, in this embodiment, the blood level in the medium to deep layer of a mucous membrane as ln(R/G) from the green image signal G (green pixels) and the red image signal R (red pixels) of the narrowband light image.

The conversion table 78 is used to effect a conversion such that as the value of the hemoglobin index rRG increases, a red color difference signal Cr increases while a blue color difference signal Cb decreases.

The enhanced color difference signal calculator 80 uses the conversion table 78 to calculate enhanced color difference signals (including color difference signals Cr and Cb) where red is enhanced according to the value of the hemoglobin index rRG. According to this embodiment, the enhanced color difference signal calculator 80 calculates enhanced color difference signals where red is increasingly enhanced as the value of the hemoglobin index rRG increases.

The image signal converter 82 produces image signals of an endoscopic image for display, which in this embodiment are image signals R, G, and B, from the enhanced luminance signal Y and the enhanced color difference signals (Cr and Cb).

The normal light image signal and the narrowband light image signal for display are stored in the storage unit 72 by unit of, for example, one sheet (frame) of image.

The normal light image signal and the narrowband light image signal outputted from the image processor 70 are inputted to the controller 68. The controller 68 causes one of the normal light image and the narrowband light image to be displayed on the monitor 18 based on the normal light image signal and the narrowband light image signal for display according to the observation mode.

Next, the operation of the endoscopic diagnosis system 10 will be described.

In the normal light observation mode, the light source controller 22 controls the laser light source LD1 to be turned off and the laser light source LD2 to be turned on. The laser beam having a central wavelength of 445 nm emitted from the laser light source LD2 illuminates the fluorescent bodies 54A, 54B, and the white light emitted from the fluorescent bodies 54A, 54B illuminate the subject, whereupon the reflected light thereof are received by the image sensor 58 to acquire the normal light image.

The imaging signal (analog signal) of the normal light image outputted from the image sensor 58 is converted into an image signal (digital signal) by the A/D converter 62 and undergoes a given image processing suited to the normal light image by the normal light image processor 70A of the image processor 68 according to the observation mode, whereupon a normal light image signal for display is outputted. Then, the controller 64 causes the normal light image corresponding to the normal light image signal for display to be displayed on the monitor 18.

On the other hand, in the narrowband light observation mode, the light source controller 22 controls both the laser light sources LD1, LD2 to be turned on. The laser beam having a central wavelength of 405 nm emitted from the laser light source LD1 and the white light excited by the laser beam, which is emitted from the laser light source LD2 and has a central wavelength of 445 nm, and emitted from the fluorescent bodies 54A, 54B are allowed to simultaneously illuminate the subject at a given emission ratio, whereupon the reflected light thereof is received by the image sensor 58 to acquire the narrowband light image.

The imaging signal (analog signal) of the narrowband light image outputted from the image sensor 58 is converted into an image signal (digital signal) by the A/D converter 62 and undergoes a given image processing suited to the narrowband light image by the narrowband light image processor 70B of the image processor 68 according to the observation mode, whereupon a narrowband light image signal for display is outputted. Then, the controller 64 causes a narrowband light image corresponding to the narrowband light image signal for display to be displayed on the monitor 18.

Image processing in the narrowband light observation mode will now be described.

The narrowband light image processor 70B first uses a formula (1) below to linearly combine the image signal of the blue pixels and the image signal of the green pixels among the image signals of blue, green and red pixels of the narrowband light image through the enhanced luminance signal calculator 74 in order to calculate the enhanced luminance signal Y.

$$Y = b*B + g*G \qquad (1)$$

In the formula, b and g are weighting factors. Weighting is made so that, for example, the ratio of the blue pixel components and the green pixel components is 7:3 in the enhanced luminance signal Y. Because the blue pixel component represents information on blood vessels in the superficial layer, the blood vessels in the superficial layer can be enhanced by increasing the weight of the blue pixel component. Further, the green pixel component may optionally be included in the calculation of the enhanced luminance signal Y to include information on the blood vessels in the medium to deep layer.

Subsequently, the hemoglobin index calculator 76 calculates the hemoglobin index rRG using the following formula (2) from the image signals of the blue, green, and red pixels of the narrowband light image.

$$rRG = \ln(R/G) \qquad (2)$$

The value of the hemoglobin index rRG increases as the hemoglobin density (blood level) in the medium to deep layer increases. Using this nature, an enhanced display of blood vessels in the superficial layer and display of medium to deep layer blood level distribution are simultaneously achieved.

Then, the enhanced color difference signal calculator 80 uses the conversion table 78 to calculate enhanced color difference signals from the hemoglobin index rRG. In this embodiment, the enhanced color difference signals include the red color difference signal Cr and the blue color difference signal Cb.

Fig. 6 is an example of conversion table representing a relationship between hemoglobin index and color-difference signal. In the figure, the vertical axis shows color difference signal (or correction value described later); the horizontal axis shows hemoglobin index rRG. According to this conversion table, as the value of the hemoglobin index rRG increases, the red color difference signal Cr increases while the blue color difference signal Cb decreases as in a linear function.

Accordingly, the color difference signals Cr and Cb corresponding to the value of the hemoglobin index rRG such that the red in the narrowband light image for display is enhanced as the blood level in the medium to deep layer increases can be calculated using that conversion table. When the hemoglobin index rRG is, for example, 1.2, the color difference signals Cr and Cb are 50 and -50, respectively.

Subsequently, the image signal converter 82 converts the enhanced luminance signal Y calculated as above and the enhanced color difference signals, i.e., color difference signals Cr and Cb, into red, green, and blue image signals to produce the narrowband light image signal for display.

Thus, in the narrowband light image corresponding to the narrowband light image signal for display, the blood level in the medium to deep layer is represented by a gradation in red of the image so that the red is increasingly enhanced as the hemoglobin index rRG increases, that is, as the blood level in the medium to deep layer increases. Because the luminance signal is produced with the weight placed on the image signal of blue pixels, the blood vessels in the superficial layer are represented in a high contrast (with a low luminance value).

Undifferentiated early-stage stomach cancers are characterized by a low blood vessel density in the tumor region as compared with well-differentiated cancers. Thus, as described above, simultaneously displaying the information on a mucous membrane medium-to-deep layer blood amount distribution and enhanced superficial-layer blood vessels in endoscopic observation compensates for a disadvantage of the narrowband light observation that discovery of a site of lesion or determination of the range of an affected region is difficult with a mere inspection into the superficial layer of a mucous membrane and enables improved diagnosis of a region affected by a tumor including the undifferentiated type.

While the medium-to-deep layer blood level is displayed in pseudo color in the above embodiment, the medium-to-deep layer blood level may be displayed by, for example, using the narrowband light image as reference image.

In this case, the red color difference signal Cr and the blue color difference signal Cb are calculated first using the following formulae (3) from the narrowband light images of the blue, green, and red pixels.

$$Cr = Y - R, \quad Cb = Y - B \qquad (3)$$

Then, color difference signals Cr and Cb in the table shown in Fig. 6, for example, are added as correction values to the color difference signals Cr and Cb calculated above. Similarly, the luminance signal Y and the color difference signals Cr and Cb are converted into the red, green, and blue image signals and displayed.

Thus, similar effects as in pseudo color display can be produced. Where the medium-to-deep layer blood level is displayed in pseudo color, the values of the color difference signals Cr and Cb decrease and the display gradually approaches a monochromatic display as the value of the hemoglobin index rRG decreases. On the other hand, by using the narrowband light image as reference image, display of an image with an enhanced red using the color of the narrowband light image as reference is possible even when the blood level is low.

Next, a second embodiment will be described.

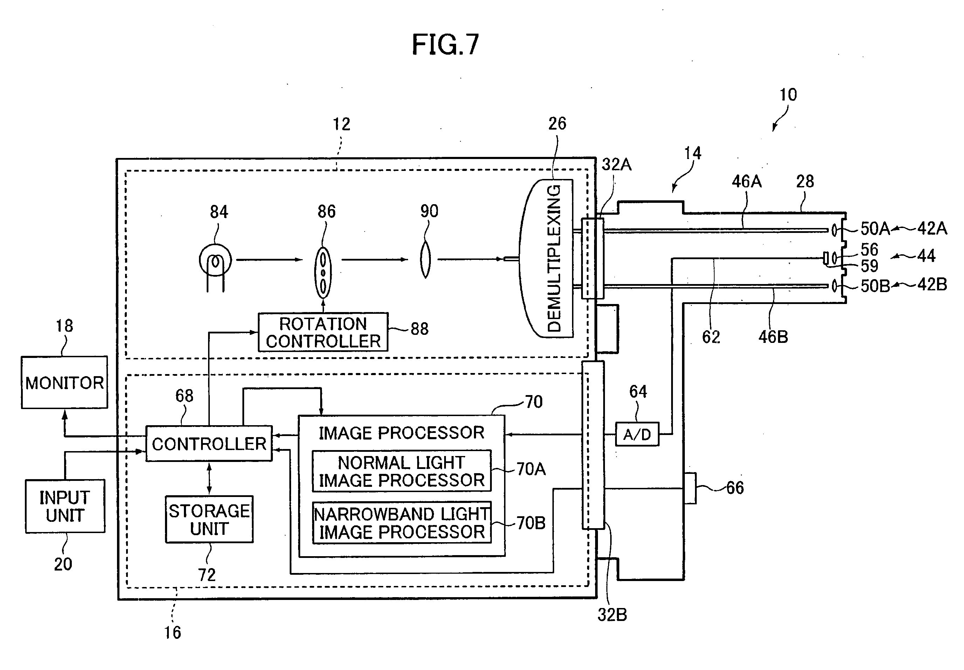

Fig. 7 is a block diagram of the second embodiment illustrating an internal configuration of the endoscopic diagnosis system shown in Fig. 1. The light source device 12 of the endoscopic diagnosis system illustrated in that drawing comprises a white light source 84, a narrowband filter 86, a rotation controller 88, a lens 90, and the coupler 26.

The white light source 84 may, for example, be in operation and emits white light whenever the light source device 12 is in operation. Examples of the white light source 84 include white light emitting lamps such as a xenon lamp, a fluorescent lamp, and a mercury lamp as well as any other light source that emits white light.

The narrowband filter 86 is a band pass filter that filters the white light emitted from the white light source 84 to pass light having a given wavelength range. The narrowband filter 86 has the shape of a disk and comprises a first to a third light filtering portion that pass first narrowband light having a wavelength of 415 nm +/- 10 nm of blue light, second narrowband light having a wavelength of 540 nm to 580 nm of green light, and third narrowband light having a wavelength of 590 nm to 700 nm of red light. The wavelength range of 415 nm +/- 10 nm of the first narrowband light is a wavelength range where hemoglobin in the blood has its greatest light absorption coefficient. The narrowband filter 86 is provided on the optical path between the white light source 84 and the lens 90 in a perpendicular position with respect to the optical path and rotated as necessary by a motor, not shown, under the control by the rotation controller 88.

The rotation controller 88 controls the rotation of the narrowband filter 86 under the control by the controller 64 of the processor 16. According to this embodiment of the endoscopic diagnosis system, a blue, a green, and a red endoscopic image are acquired by frame sequential method wherein three frames make up one set of imaging period. In the first to the third frame constituting one set of imaging period, the rotation controller 88 controls the rotation of the narrowband filter 86 so that the first to the third light filtering portion are sequentially aligned with the optical path in each frame.

On the inside of the illumination window 42A of the endoscope device 14 is provided an optical fiber 46A having an optical system including, for example, a lens 50A at the tip portion; on the inside of the illumination window 42B is likewise provided an optical fiber 46B having an optical system including, for example, a lens 50B at the tip portion. On the inside of the observation window 44 is installed an optical system including, for example, an object lens unit 56; behind the object lens unit 56 is provided an image sensor 59. The image sensor 59 of this embodiment is a monochromatic CCD image sensor.

The processor 16 has the same configuration as in the first embodiment.

Next, the operation of the second embodiment of the endoscopic diagnosis system will be described.

According to this embodiment of the endoscopic diagnosis system, the blue, the green, and the red endoscopic image are acquired by frame sequential method wherein three frames make up one set of imaging period.

In the light source device, the rotation controller 88 controls the rotation of the narrowband filter 86 so that the first to the third light filtering portion are sequentially aligned with the optical path in each frame of the first to the third frame constituting one set of imaging period. Thus, the first to the third narrowband light sequentially pass through the narrowband filter 86 in each frame, are condensed by the lens 90, and divided into two channels of light through the coupler 26 before being transmitted to the connector portion 32A.

In the endoscope device 14, the first to the third narrowband light emitted from the light source device 12 are guided by the optical fibers 46A, 46B to illuminate the subject's region under observation, and the reflected light thereof are imaged by the image sensor 59. The image sensor 59 sequentially outputs imaging signals of the blue, green, and red endoscopic images having luminances corresponding to the reflected light of the first to the third narrowband light, and these imaging signals are sequentially converted into image signals of the blue, the green, and the red endoscopic image by the A/D converter 62.

The operations to follow in the normal light observation mode and the narrowband light observation mode are all the same as in the first embodiment.

Accordingly, in the normal light observation mode, the normal light image corresponding to the image signals of the blue, the green, and the red endoscopic image is displayed on the monitor 18.

In the narrowband light observation mode, on the other hand, the enhanced luminance signal Y is calculated from the blue and the green endoscopic image; the hemoglobin index rRG is calculated from the image signals of the green and the red endoscopic image. Then, the enhanced color difference signal is calculated from the hemoglobin index rRG using the conversion table 78, and the enhanced luminance signal Y and the enhanced color difference signals are converted into the red, green, and blue image signals to display the narrowband light image on the monitor 18.

**Claims**

1. An endoscopic diagnosis system, comprising:

   a white light source for emitting white light;
   a first narrowband light (LD1) source for emitting first narrowband light having a given wavelength range of blue light;
   an image sensor (58) including blue, green and red color filters on its light receiving surface and receiving reflected light of the white light and the first narrowband light emitted with a given emission ratio to illuminate a subject from the subject in a narrowband light observation mode to acquire a narrowband light image,
an image processor (70) for calculating an enhanced luminance signal Y where a blue image signal B of the narrowband light image is enhanced, calculating a hemoglobin index representing a blood level in a medium to deep layer of a mucous membrane from a green image signal G and a red image signal R of the narrowband light image, and producing an image signal of an endoscopic image for display producing an image signal of an endoscopic image for display from the enhanced luminance signal Y and the enhanced color difference signal; and
a monitor (18) for displaying an endoscopic image corresponding to the image signal of the endoscopic image for display **characterized in that** the image processor is adapted for calculating an enhanced color difference signal including a red color difference signal Cr and a blue color difference signal Cb and enhancing red according to a value of the hemoglobin index.

2. The endoscopic diagnosis system according to Claim 1, wherein the image processor is adapted to (70) calculate the enhanced luminance signal Y by weighting the blue image signal B and the green image signal G with a given ratio.

3. The endoscopic diagnosis system according to Claim 1 or 2, wherein the image processor (70) is adapted to calculate the hemoglobin index from the green image signal G and the red image signal B as ln(R/G).

4. The endoscopic diagnosis system according to any one of Claims 1 to 3, wherein the image processor (70) is adapted to calculate the enhanced color difference signal by using a conversion table (78) according to which a value of a red color difference signal increases and a value of a blue color difference signal decreases as the value of the hemoglobin index increases.

5. The endoscopic diagnosis system according to any one of Claims 1 to 3, wherein the image processor (70) is adapated to calculate a blue color difference signal representing a difference between the enhanced luminance signal Y and the blue image signal B and a red color difference signal representing a difference between the enhanced luminance signal Y and the red image signal R, to use a conversion table according to which a red correction value increases and a blue correction value decreases as the value of the hemoglobin index increases to add up the blue color difference signal and the blue correction value and add up the red color difference signal and the red correction value, and thereby to calculate the enhanced color difference signal.

6. The endoscopic diagnosis system according to any one of Claims 1 to 5, wherein the first narrowband light (LD1) has a given wavelength range within 395 nm to 455 nm.

7. The endoscopic diagnosis system according to Claim 6, wherein the first narrowband light (LD1) has a given wavelength range of 405 nm +/- 10 nm.

8. The endoscopic diagnosis system according to any one of Claims 1 to 5, wherein the white light source comprises a second narrowband light source (LD2) for emitting second narrowband light having a given wavelength range of blue light and a fluorescent body for emitting excited luminescence light when illuminated by the second narrowband light, so that the second narrowband light and the excited luminescence light are adapted to generate pseudo white light.

9. The endoscopic diagnosis system according to Claim 8, wherein the second narrowband light (LD2) has a wavelength range of 445 nm +/-10 nm.

10. The endoscopic diagnosis system according to Claim 8 or 9, wherein the first narrowband light (LD1) and the second narrowband light (LD2) have a wavelength range of 445 nm +/- 10 nm, and wherein the white light source is adapted to use the first narrowband light (LD1) source as the second narrowband light source (LD2).

11. An endoscopic diagnosis system, comprising:

a white light source (84) for emitting white light;
color filters (86) for separating the white light into narrowband light, green light and red light, the narrowband light having a given wavelength range of blue light;
an image sensor (58) for receiving reflected light of the narrowband light having the given wavelength range of the blue light, the green light and the red light emitted by frame sequential method to illuminate a subject and sequentially acquiring a blue narrowband light image, a green normal light image and a red normal light image in a narrowband light observation mode;
an image processor for calculating an enhanced luminance signal Y where an image signal B of the blue narrowband light image is enhanced, calculating a hemoglobin index representing blood level in a medium to deep layer of a mucous membrane from an image signal G of the green normal light image and an image signal R of the red normal light image, and producing an image signal of an endoscopic image for display from the enhanced luminance signal and the enhanced color difference signal; and
a monitor (18) for displaying an endoscopic image corresponding to the image signal of the endoscopic image for display **characterized in that** the image processor is adapted for calculating an enhanced color difference signal where red is enhanced according to a value of the hemoglobin index.

12. The endoscopic diagnosis system according to Claim 11, wherein the image processor (70) calculate the enhanced luminance signal Y by weighting the image signal B of the blue narrowband light image and the image signal G of the green normal light image with a given ratio.

13. The endoscopic diagnosis system according to Claim 11 or 12, wherein the image processor (70) calculate the hemoglobin index as ln(R/G) from the image signal G of the green normal light image and the image signal R of the red normal light image.

14. The endoscopic diagnosis system according to any one of Claims 11 to 13, wherein the image processor (70) calculate the enhanced color difference signal by using a conversion table (78) according to which a value of a red color difference signal increases and a value of a blue color difference signal decreases as the value of the hemoglobin index increases.

15. The endoscopic diagnosis system according to any one of Claims 11 to 13, wherein the image processor (70) is adapted to calculate a blue color difference signal representing a difference between the enhanced luminance signal and the image signal B of the blue normal light image and a red color difference signal representing a difference between the enhanced luminance signal and the image signal R of the red normal light image, uses a conversion table (78) according to which a red correction value increases and a blue correction value decreases as the value of the hemoglobin index increases to add up the blue color difference signal and the blue correction value and add up the red color difference signal and the red correction value, and thereby calculates the enhanced color difference signal.

16. The endoscopic diagnosis system according to any one of Claims 11 to 15, wherein one of the color filters (86) for separating the blue narrowband light from the white light is adapted to separate narrowband light having a wavelength range of 415 nm +/- 10 nm from the white light.

**Patentansprüche**

1. Endoskopisches Diagnosesystem, umfassend:

eine Weißlichtquelle zum Emittieren von weißem Licht;
eine erste schmalbandige Lichtquelle (LD1) zum Emittieren ersten schmalbandigen Lichts mit einem gegebenen Wellenlängenbereich von blauem Licht;
einen Bildsensor (58), der auf seiner Lichtempfangsfläche Farbfilter für Blau, Grün und Rot enthält und reflektiertes Licht des Weißlichts und des ersten schmalbandigen Lichts, die mit einem gegebenen Emissionsverhältnis zum Beleuchten eines Subjekts emittiert werden, von dem Subjekt in einem Schmalbandlicht-Betrachtungsmodus empfängt, um ein Schmalbandlicht-Bild zu erfassen,
einen Bildprozessor (70) zum Berechnen eines verstärkten Luminanzsignals Y, wobei ein blaues Bildsignal B des Schmalbandlicht-Bilds verstärkt wird zum Berechnen eines Hämoglobin-Index', der einen Blutpegel in einer mittleren bis tiefen Schicht einer Schleimhaut aus einem grünen Bildsignal G und einem roten Bildsignal R des Schmalbandlicht-Bilds repräsentiert, und zum Erzeugen eines Bildsignals eines endoskopischen Bilds für die Anzeige aus dem verstärkten Luminanzsignal Y und dem verstärkten Farbdifferenzsignal; und
einen Monitor (18) zum Anzeigen eines endoskopischen Bilds entsprechend dem Bildsignal des endoskopischen Bilds für die Anzeige, **dadurch gekennzeichnet, dass** der Bildprozessor dazu ausgebildet ist, ein verstärktes Farbdifferenzsignal mit einem roten Farbdifferenzsignal Cr und einem blauen Farbdifferenzsignal Cb zu berechnen und Rot nach Maßgabe des Werts des Hämoglobin-Index' zu verstärken.

2. Endoskopisches Diagnosesystem nach Anspruch 1, bei dem der Bildprozessor dazu ausgebildet ist, das verstärkte Luminanzsignal Y zu berechnen, indem er das blaue Bildsignal B und das grüne Bildsignal G in einem gegebenen Verhältnis wichtet.

3. Endoskopisches Diagnosesystem nach Anspruch 1 oder 2, bei dem der Bildprozessor dazu ausgebildet ist, den Hämoglobin-Index zu berechnen aus dem grünen Bildsignal G und dem roten Bildsignal B als ln (R/G).

4. Endoskopisches Diagnosesystem nach einem der Ansprüche 1 bis 3, bei dem der Bildprozessor (70) dazu ausgebildet ist, das verstärkte Farbdifferenzsignal zu berechnen unter Verwendung einer Umwandlungstabelle (78), gemäß der ein Wert eines roten Farbdifferenzsignals zunimmt und ein Wert eines blauen Farbdifferenzsignals abnimmt, wenn der Wert des Hämoglobin-Index' zunimmt.

5. Endoskopisches Diagnosesystem nach einem der Ansprüche 1 bis 3, bei dem der Bildprozessor (70) dazu ausgebildet ist, ein blaues Farbdifferenzsignal, das eine Differenz zwischen dem verstärkten Luminanzsignal Y und dem blauen Bildsignal B repräsentiert, und ein rotes Differenzsignal, welches eine Differenz zwischen dem verstärkten Luminanzsignal Y und dem roten Bildsignal R repräsentiert, zu berechnen, eine Umwandlungstabelle zu verwenden, gemäß der ein roter Korrekturwert zunimmt und ein blauer Korrekturwert abnimmt, wenn der Wert des Hämoglobin-Index' zunimmt, und das blaue Farbdifferenzsignal und den blauen Korrekturwert zu addieren, und das rote Farbdifferenzsignal und den roten Korrekturwert zu addieren, um dadurch das verstärkte Farbdifferenzsignal zu berechnen.

6. Endoskopisches Diagnosesystem nach einem der Ansprüche 1 bis 5, bei dem das erste Schmalbandlicht (LD1) einen gegebenen Wellenlängenbereich von 395 nm bis 455 nm aufweist.

7. Endoskopisches Diagnosesystem nach Anspruch 6, bei dem das erste Schmalbandlicht (LD1) einen gegebenen Wellenlängenbereich von 405 nm +/- 10 nm aufweist.

8. Endoskopisches Diagnosesystem nach einem der Ansprüche 1 bis 5, bei dem die Weißlichtquelle eine zweite Schmalbandlicht-Quelle (LD2) zum Emittieren eines zweiten schmalbandigen Lichts mit einem gegebenen Wellenlängenbereich blauen Lichts und einen Fluoreszenzkörper zum Emittieren angeregten Lumineszenzlichts bei Beleuchtung durch das zweite Schmalbandlicht aufweist, so dass das zweite Schmalbandlicht und das angeregte Lumineszenzlicht geeignet sind, Pseudo-Weißlicht zu erzeugen.

9. Endoskopisches Diagnosesystem nach Anspruch 8, bei dem das zweite Schmalbandlicht (LD2) einen Wellenlängenbereich von 445 nm +/- 10 nm besitzt.

10. Endoskopisches Diagnosesystem nach Anspruch 8 oder 9, bei dem das erste Schmalbandlicht (LD1) und das zweite Schmalbandlicht (LD2) einen Wellenlängenbereich von 445 nm +/- 10 nm besitzen, wobei die Weißlichtquelle dazu ausgebildet ist, die erste Schmalbandlichtquelle (LD1) als die zweite Schmalbandlichtquelle (LD2) zu verwenden.

11. Endoskopisches Diagnosesystem, umfassend:

   eine Weißlichtquelle (84) zum Emittieren von weißem Licht;
   Farbfilter (86) zum Separieren des Weißlichts in Schmalbandlicht, grünes Licht und rotes Licht, wobei das Schmalbandlicht einen gegebenen Wellenlängenbereich blauen Lichts aufweist;
   einen Bildsensor (58) zum Empfangen reflektierten Lichts des Schmalbandlichts mit dem gegebenen Wellenlängenbereich des blauen Lichts, des grünen Lichts und des roten Lichts, welches nach einem Vollbild-sequentiellen Verfahren emittiert wird, um ein Subjekt zu beleuchten, und zum sequentiellen Erfassen eines blauen Schmalbandlicht-Bilds, eines grünen Normallicht-Bilds und eines roten Normallicht-Bilds in einem Schmalbandlicht-Betrachtungsmodus;
   einen Bildprozessor zum Berechnen eines verstärkten Luminanzsignals Y, wobei ein Bildsignal B des blauen Schmalbandlicht-Bilds verstärkt ist zum Berechnen eines Hämoglobin-Index', der einen Blutpegel in einer mittleren bis tiefen Schicht eines Schleimhaut anhand eines Bildsignals G eines grünen Normallicht-Bilds und eines Bildsignals R des roten Normallicht-Bilds repräsentiert, und zum Erzeugen eines Bildsignals aus einem endoskopischen Bild für eine Anzeige aus dem verstärkten Luminanzsignal Y und dem verstärkten Farbdifferenzsignal; und
   einen Monitor (18) zum Anzeigen eines endoskopischen Bilds entsprechend dem Bildsignal des endoskopischen Bilds für eine Anzeige, **dadurch gekennzeichnet, dass** der Bildprozessor dazu ausgebildet ist, ein verstärktes Farbdifferenzsignal, welches ein rotes Farbdifferenzsignal Cr und ein blaues Farbdifferenzsignal Cb enthält, zu berechnen, wobei Rot nach Maßgabe eines Werts des Hämoglobin-Index' verstärkt ist.

12. Endoskopisches Diagnosesystem nach Anspruch 11, bei dem der Bildprozessor dazu ausgebildet ist, das verstärkte Luminanzsignal Y dadurch zu berechnen, dass er das Bildsignal B des blauen Schmalbandlicht-Bilds und das Bildsignal G des grünen Normallicht-Bilds mit einem gegebenen Verhältnis wichtet.

13. Endoskopisches Diagnosesystem nach Anspruch 11 oder 12, bei dem der Bildprozessor (70) dazu ausgebildet ist, den Hämoglobin-Index als In (R/G) aus dem Bildsignal G des grünen Normallicht-Bilds und dem Bildsignal R des roten Normallicht-Bilds zu berechnen.

14. Endoskopisches Diagnosesystem nach einem der

**21**      **EP 2 474 265 B1**      **22**

Ansprüche 11 bis 13, bei dem der Bildprozessor (70) dazu ausgebildet ist, das verstärkte Farbdifferenzsignal zu berechnen unter Verwendung einer Umwandlungstabelle (78), gemäß der ein Wert eines roten Farbdifferenzsignals zunimmt und ein Wert eines blauen Farbdifferenzsignals abnimmt, wenn der Wert des Hämoglobin-Index' zunimmt.

**15.** Endoskopisches Diagnosesystem nach einem der Ansprüche 11 bis 13, bei dem der Bildprozessor (70) dazu ausgebildet ist, ein blaues Farbdifferenzsignal, welches eine Differenz zwischen dem verstärkten Luminanzsignal und dem Bildsignal B des blauen Normallicht-Bilds repräsentiert, und ein rotes Farbdifferenzsignal, welches eine Differenz zwischen dem verstärkten Luminanzsignal und dem Bildsignal R des roten Normallicht-Bilds repräsentiert, zu berechnen, eine Umwandlungstabelle (78) verwendet, gemäß der ein roter Korrekturwert zunimmt und ein blauer Korrekturwert abnimmt, wenn der Wert des Hämoglobin-Index' zunimmt, und das blaue Farbdifferenzsignal auf den blauen Korrekturwert addiert, und das rote Farbdifferenzsignal auf den roten Korrekturwert addiert, um dadurch das verstärkte Farbdifferenzsignal zu berechnen.

**16.** Endoskopisches Diagnosesystem nach einem der Ansprüche 11 bis 15, bei dem einer der Farbfilter (86) zum Separieren des blauen schmalbandigen Lichts von dem Weißlicht dazu ausgebildet ist, das Schmalbandlicht mit einem Wellenlängenbereich von 415 nm +/- 10 nm von dem Weißlicht zu separieren.

**Revendications**

**1.** Système de diagnostic endoscopique comprenant :

une source de lumière blanche pour émettre de la lumière blanche ;
une première source de lumière à bande étroite (LD1) pour émettre une première lumière à bande étroite ayant une plage de longueurs d'onde donnée de lumière bleue ;
un capteur d'image (58) incluant des filtres de couleur bleue, verte et rouge sur sa surface réceptrice de lumière et recevant du sujet la lumière réfléchie de la lumière blanche et de la première lumière à bande étroite émise avec un rapport d'émission donné pour éclairer un sujet dans un mode d'observation de lumière à bande étroite pour acquérir une image de lumière à bande étroite,
un processeur d'image (70) pour calculer un signal de luminance amélioré Y où un signal d'image bleue B de l'image de lumière à bande étroite est amélioré, calculer l'indice d'hémoglobine représentant le niveau de sang dans une couche moyenne à profonde d'une membrane de muqueuse à partir d'un signal d'image verte G et d'un signal d'image rouge R de l'image de lumière à bande étroite, et produire un signal d'image d'une image endoscopique pour affichage à partir du signal de luminance amélioré Y et du signal de différence de couleur amélioré ; et
un moniteur (18) pour afficher une image endoscopique correspondant au signal d'image de l'image endoscopique pour affichage, **caractérisé en ce que** le processeur d'image est adapté à calculer un signal de différence de couleur amélioré incluant un signal de différence de couleur rouge Cr et un signal de différence de couleur bleue Cb de rouge amélioré et améliorant le rouge en fonction de la valeur de l'indice d'hémoglobine.

**2.** Système de diagnostic endoscopique selon la revendication 1, dans lequel le processeur d'image (70) est adapté à calculer le signal de luminance amélioré Y par pondération du signal d'image bleue B et du signal d'image verte G avec un rapport donné.

**3.** Système de diagnostic endoscopique selon la revendication 1 ou 2, dans lequel le processeur d'image (70) est adapté à calculer l'indice d'hémoglobine à partir du signal d'image verte G et du signal d'image rouge B par ln(R/G).

**4.** Système de diagnostic endoscopique selon l'une quelconque des revendications 1 à 3, dans lequel le processeur d'image (70) est adapté à calculer le signal de différence de couleur amélioré en utilisant une table de conversion (78) selon laquelle la valeur d'un signal de différence de couleur rouge augmente et la valeur d'un signal de différence de couleur bleue diminue à mesure que la valeur de l'indice d'hémoglobine augmente.

**5.** Système de diagnostic endoscopique selon l'une quelconque des revendications 1 à 3, dans lequel le processeur d'image (70) est adapté à calculer un signal de différence de couleur bleue représentant la différence entre le signal de luminance amélioré Y et le signal d'image bleue B et un signal de différence de couleur rouge représentant la différence entre le signal de luminance amélioré Y et le signal d'image rouge R, à utiliser une table de conversion selon laquelle la valeur de correction de rouge augmente et la valeur de correction de bleue diminue à mesure que la valeur de l'indice d'hémoglobine augmente, pour ajouter le signal de différence de couleur bleue et la valeur de correction de bleue et ajouter le signal de différence de couleur rouge et la valeur

**12**

de correction de rouge, et à calculer ainsi le signal de différence de couleur amélioré.

**6.** Système de diagnostic endoscopique selon l'une quelconque des revendications 1 à 5, dans lequel la première lumière à bande étroite (LD1) possède une plage de longueurs d'onde donnée entre 395 nm et 455 nm.

**7.** Système de diagnostic endoscopique selon la revendication 6, dans lequel la première lumière à bande étroite (LD1) possède une plage de longueurs d'onde donnée de 405 nm +/-10 nm.

**8.** Système de diagnostic endoscopique selon l'une quelconque des revendications 1 à 5, dans lequel la source de lumière blanche comprend une seconde source de lumière à bande étroite (LD2) pour émettre une seconde lumière à bande étroite ayant une plage de longueurs d'onde donnée de lumière bleue et un corps fluorescent pour émettre de la lumière de luminescence excitée lorsqu'il est éclairé par la seconde lumière à bande étroite, de sorte que la seconde lumière à bande étroite et la lumière de luminescence excitée sont adaptées à générer une pseudo lumière blanche.

**9.** Système de diagnostic endoscopique selon la revendication 8, dans lequel la seconde lumière à bande étroite (LD2) possède une plage de longueurs d'onde de 445 nm +/-10 nm.

**10.** Système de diagnostic endoscopique selon la revendication 8 ou 9, dans lequel la première lumière à bande étroite (LD1) et la seconde lumière à bande étroite (LD2) possèdent une plage de longueurs d'onde de 445 nm +/ 10 nm et dans lequel la source de lumière blanche est adaptée à utiliser la première source de lumière à bande étroite (LD1) en tant que seconde source de lumière à bande étroite (LD2).

**11.** Système de diagnostic endoscopique comprenant :

une source de lumière blanche (84) pour émettre de la lumière blanche ;
des filtres de couleur (86) pour séparer la lumière blanche en lumière à bande étroite, lumière verte et lumière rouge, la lumière à bande étroite ayant une plage de longueurs d'onde donnée de lumière bleue ;
un capteur d'image (58) pour recevoir la lumière réfléchie de la lumière à bande étroite ayant la plage de longueurs d'onde donnée de la lumière bleue, la lumière verte et la lumière rouge émises par un procédé séquentiel de trame pour éclairer un sujet et acquérir séquentiellement une image de lumière à bande étroite bleue, une image de lumière normale verte et une image de lumière normale rouge dans un mode d'observation de lumière à bande étroite ;
un processeur d'image pour calculer un signal de luminance amélioré Y où un signal d'image bleue B de l'image de lumière à bande étroite bleue est amélioré, calculer l'indice d'hémoglobine représentant le niveau de sang dans une couche moyenne à profonde d'une membrane de muqueuse à partir d'un signal d'image G de l'image de lumière normale verte et d'un signal d'image R de l'image de lumière normale rouge, et produire un signal d'image d'une image endoscopique pour affichage à partir du signal de luminance amélioré Y et du signal de différence de couleur amélioré ; et
un moniteur (18) pour afficher une image endoscopique correspondant au signal d'image de l'image endoscopique pour affichage, **caractérisé en ce que** le processeur d'image est adapté à calculer un signal de différence de couleur amélioré incluant un signal de différence de couleur rouge Cr et un signal de différence de couleur bleue Cb de rouge amélioré et où le rouge est amélioré en fonction de la valeur de l'indice d'hémoglobine.

**12.** Système de diagnostic endoscopique selon la revendication 11, dans lequel le processeur d'image (70) est adapté à calculer le signal de luminance amélioré Y par pondération du signal d'image B de l'image de lumière à bande étroite bleue et du signal d'image G de l'image de lumière normale verte avec un rapport donné.

**13.** Système de diagnostic endoscopique selon la revendication 11 ou 12, dans lequel le processeur d'image (70) est adapté à calculer l'indice d'hémoglobine par ln(R/G) à partir du signal d'image G de l'image de lumière normale verte et du signal d'image R de l'image de lumière normale rouge.

**14.** Système de diagnostic endoscopique selon l'une quelconque des revendications 11 à 13, dans lequel le processeur d'image (70) est adapté à calculer le signal de différence de couleur amélioré en utilisant une table de conversion (78) selon laquelle la valeur d'un signal de différence de couleur rouge augmente et la valeur d'un signal de différence de couleur bleue diminue à mesure que l'indice d'hémoglobine augmente.

**15.** Système de diagnostic endoscopique selon l'une quelconque des revendications 11 à 13, dans lequel le processeur d'image (70) est adapté à calculer un signal de différence de couleur bleue représentant la différence entre le signal de luminance amélioré et le signal d'image B de l'image de lumière normale bleue et un signal de différence de couleur rouge représentant la différence entre le signal de luminance amélioré et le signal d'image R de l'image de lumière normale rouge, à utiliser une table de conversion (78) selon laquelle la valeur de correction de rouge augmente et la valeur de correction de bleue diminue à mesure que la valeur de l'indice d'hémoglobine augmente pour ajouter le signal de différence de couleur bleue et la valeur de correction de bleue et ajouter le signal de différence de couleur rouge et la valeur de correction de rouge, et à calculer ainsi le signal de différence de couleur amélioré.

16. Système de diagnostic endoscopique selon l'une quelconque des revendications 11 à 15, dans lequel l'un des filtres de couleur (86) pour séparer la lumière à bande étroite bleue de la lumière blanche est adapté à séparer de la lumière blanche la lumière à bande étroite ayant une plage de longueurs d'onde de 415 nm +/-10 nm.

# FIG.1

# FIG.2

# FIG.3

445nm LASER BEAM

EXCITED LUMINESCENCE
LIGHT OF FLUORESCENT BODY } WHITE LIGHT

EMISSION INTENSITY

445    500        600        700

WAVELENGTH(nm)

# FIG.4

TRANSMITTANCE(%)

100

B        G        R

50

400    500    600    700

WAVELENGTH(nm)

# FIG.5

70B

ENHANCED LUMINANCE
SIGNAL CALCULATOR — 74

B

HEMOGLOBIN INDEX
CALCULATOR — 76

G, R

ENHANCED COLOR
DIFFERENCE SIGNAL
CALCULATOR — 80

CONVERSION
TABLE — 78

IMAGE SIGNAL OF
NARROWBANDLIGHT
IMAGE FOR DISPLAY ← IMAGE SIGNAL CONVERTER — 82

# FIG.6

# FIG.7

EP 2 474 265 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3559755 B

- EP 1302152 A